# EUROPÄISCHE PATENTSCHRIFT

(11) **EP 0 789 019 B1**
(45) Veröffentlichungstag und Bekanntmachung des Hinweises auf die Patenterteilung: **13.06.2001**
(21) Anmeldenummer: 97102004.5
(22) Anmeldetag: 07.02.1997
(51) Int. Cl.: C07D 233/70

(54) **Verfahren zur Herstellung von 4-Oxoimidazoliniumsalzen**
Process for the production of 4-oxoimidazolinium salts
Procédé pour la préparation de sels du 4-oxoimidazolinium

(30) Priorität: 09.02.1996 CH 33296
(43) Veröffentlichungstag der Anmeldung: 13.08.1997
(73) Patentinhaber: LONZA AG, CH-3945 Gampel/Wallis (CH)
(72) Erfinder: Jackson, Barry, Dr., 3902 Brig/Glis (Kanton Wallis) (CH)
(74) Vertreter: Winter, Brandl, Fürniss, Hübner, Röss, Kaiser, Polte Partnerschaft

(56) Entgegenhaltungen:
- EP-A- 0 205 879
- WO-A-91/14679
- US-A- 5 424 450
- TETRAHEDRON, Bd. 40, Nr. 12, 1984, Seiten 2395-2404, XP002032284 VERSCHAVE ET AL: "N-Acylated alpha-Aminonitriles and their conversion into 5-Aminoazole,5(4H)-Oxazolone and 4(5H)-Imidazolone derivatives"

## Beschreibung

Die vorliegende Erfindung betrifft ein Verfahren zur Herstellung von 4-Oxoimidazoliniumsalzen der allgemeinen Formel 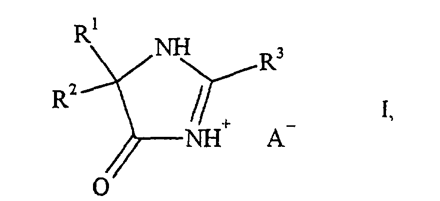
worin R¹ und R² entweder unabhängig voneinander C₁₋₁₀-Alkyl, C₂₋₁₀-Alkenyl, C₃₋₇-Cycloalkyl oder gegebenenfalls substituiertes Aryl, Arylalkyl, Heteroaryl oder Heteroarylalkyl bedeuten oder R¹ und R² zusammen mit dem verbindenden Kohlenstoffatom einen drei- bis siebengliedrigen gesättigten oder ungesättigten carbocyclischen oder heterocyclischen Ring bilden,
R³ eine C₁₋₁₀-Alkylgruppe, C₂₋₁₀-Alkenylgruppe, C₃₋₇-Cycloalkylgruppe, eine gegebenenfalls substituierte Arylgruppe, Arylalkylgruppe oder Heteroarylgruppe
und A⁻ ein Anion einer starken Säure aus der Gruppe bestehend aus den Halogenwasserstoffen, Schwefelsäure, Ameisensäure, Methansulfonsäure und Trifluoressigsäure ist.
Unter C₁₋₁₀-Alkylgruppen sind hier und im folgenden sowohl lineare als auch verzweigte primäre, sekundäre und tertiäre Alkylgruppen mit 1 bis 10 Kohlenstoffatomen zu verstehen. Entsprechend sind unter C₂₋₁₀-Alkenylgruppen sowohl lineare als auch verzweigte primäre, sekundäre und tertiäre Kohlenwasserstoffreste mit einer oder mehreren Doppelbindungen in beliebiger Position zu verstehen. Unter Arylgruppen sind Phenyl und Naphthyl zu verstehen. Unter Arylalkylgruppen sind mit Arylgruppen substituierte C₁₋₄-Alkylgruppen zu verstehen, insbesondere Benzyl und Phenylethyl. Unter Heteroarylgruppen sind Furyl oder Thienyl (Thiophen-yl) zu verstehen, unter Heteroarylalkyl dementsprechend Gruppen wie Furfuryl (Furylmethyl) oder Thenyl (Thiophen-ylmethyl). Die Aryl-, Arylalkyl-, Heteroaryl- oder Heteroarylalkylgruppen können gegebenenfalls einen oder mehrere Substituenten aus der Gruppe C₁₋₄-Alkyl, C₁₋₄-Alkoxy oder Halogen tragen.

Verbindungen dieser Art, beziehungsweise die diesen Salzen zugrundeliegenden 2-Imidazolin-4-one (1*H*-Imidazol-5(4*H*)-one), sind wichtige Zwischenprodukte in der Synthese von pharmazeutischen Wirkstoffen wie zum Beispiel Angiotensin II-Antagonisten (WO-A 91/14679, US-A 5 424 450). Sie wurden bisher beispielsweise durch Acylierung von α-Aminonitrilen zu den entsprechenden Amidonitrilen (= α-Acylaminonitrilen, auch als "aliphatische Reissert-Verbindungen" bezeichnet), saure Hydrolyse der Nitrilgruppe zur Carbamoylgruppe und anschliessende basenkatalysierte Cyclisierung des so erhaltenen Amidoamids hergestellt (US-A 5 424 450, Scheme 3) Die Reihenfolge von Acylierung und Hydrolyse kann dabei auch vertauscht werden (WO-A 91/14679, S. 25-26) Ein Nachteil dieser Verfahren ist der notwendige Wechsel von sauren und basischen Reaktionsbedingungen mit einer jeweils erforderlichen Neutralisation und der Bildung entsprechend grosser Mengen von Abfallsalzen.

P. Verschave et al. (Tetrahedron **40:**2395-2404; 1986) beschreiben die Bildung von 4-Imidazolonderivaten durch Umsetzung von α-Acylaminonitrilen mit Oxalylchlorid.

Die EP-A-0 205 879 offenbart die Herstellung von 5-Oxo-imidazolinylbenzoesäuren und 5-Oxo-imidazolinylnicotinsäuren durch Cyclisierung von α-Acylaminonitrilen in mit Halogenwasserstoff gesättigten Chlorkohlenwasserstoffen unter wasserfreien Bedingungen. Die dabei gebildeten Nebenprodukte können dann anschließend über mehrere Verfahrensschritte entweder mit Wasser ebenfalls in die gewünschten Benzoe- oder Nicotinsäuren oder mit Alkoholen in die entsprechenden Ester überführt werden.

Aufgabe der vorliegenden Erfindung war daher, ein einfacheres Verfahren bereitzustellen, das weniger Abfall produziert.

Erfindungsgemass wird diese Aufgabe durch das Verfahren nach Patentanspruch 1 gelöst.

Es wurde überraschend gefunden, dass es möglich ist, aus α-Acylaminonitrilen der allgemeinen Formel 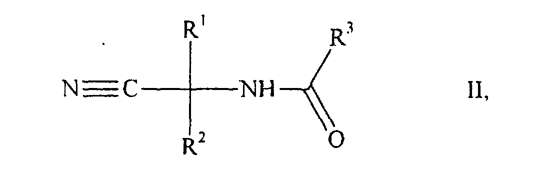 worin R¹, R² und R³ die oben genannten Bedeutungen haben, in einem nichtwassrigen Lösungsmittel in Gegenwart eines niedrigen Alkohols und einer starken Säure der allgemeinen Formel HA, worin A, beziehungsweise das Anion A⁻, die oben genannte Bedeutung hat, in einer Stufe ohne Isolation eines Zwischenprodukts und ohne Neutralisationsschritt direkt die 4-Oxoimidazoliniumsalze (I) zu erhalten

Die α-Acylaminonitrile (II) können nach bekannten Methoden hergestellt werden, beispielsweise aus den entsprechenden Carbonylverbindungen R¹-C(=O)-R² durch Umsetzung mit Blausäure und Ammoniak nach Strecker und anschliessende Acylierung des so erhaltenen α-Aminonitrils mit einem Carbonsäurechlorid R³COCl

Als starke Säure HA eignen sich sowohl anorganische Säuren ("Mineralsäuren") als auch organische Säuren aus der Gruppe bestehend aus den Halogenwasserstoffen, Schwefelsäure, Ameisensäure, Trifluoressigsäure und Methansulfonsäure. Besonders bevorzugt ist Chlorwasserstoffsäure.

Die Saure wird vorzugsweise in einer Menge von 1 bis 2 Äquivalenten, vorzugsweise von 1,1 bis 1,5 Äquivalenten pro Mol Ausgangsmaterial eingesetzt.

Als nichtwässriges Lösungsmittel wird vorzugsweise ein Lösungsmittel aus der Gruppe der aromatischen Kohlenwasserstoffe wie Benzol, Toluol oder Xylol oder aus der Gruppe der halogenierten Kohlenwasserstoffe wie beispielsweise Dichlormethan eingesetzt.

Besonders bevorzugt ist eine Ausführungsform, in der der niedrige Alkohol, d.h. Methanol, Ethanol, Propanol, Butanol und Isopropylalkohol, gleichzeitig als nichtwassriges Lösungsmittel dient.

Das erfindungsgemässe Verfahren wird vorzugsweise zur Herstellung von 4-Oxoimidazoliniumsalzen (I) eingesetzt, die Spiroverbindungen sind, in welchen R¹ und R² zusammen mit dem verbindenden Kohlenstoffatom einen Cyclopentan- oder Cyclohexanring bilden.

Ebenfalls bevorzugt ist die Herstellung von 4-Oxoimidazoliniumsalzen (I), in denen R³ eine C₁₋₆-Alkylgruppe ist.

Die Reaktionstemperatur beträgt vorteilhaft 0 bis 120 °C, vorzugsweise 20 bis 100 °C.

Selbstverständlich liegt es auch im Rahmen der Erfindung, die 4-Oxoimidazoliniumsalze (1) mit Basen in die entsprechenden Imidazolin-4-one uberzufuhren.

Die nachfolgenden Beispiele verdeutlichen die Durchfuhrung des erfindungsgemässen Verfahrens, ohne dass darin eine Einschränkung zu sehen ist.

### Beispiel 1

### 2-Butyl-1,3-diaza-spiro[4.4]non-2-en-4-on-Monohydrochlorid

### (I, R¹ + R² = -(CH₂)₄-, R³ = n-Butyl)

In 28 g wasserfreiem Ethanol wurden 6,80 g (30 mmol) *N*-(1-Cyancyclopentyl)pentanamid (hergestellt aus Cyclopentanon durch Strecker-Synthese zum 1-Aminocyclopentancarbonitril und Acylierung mit Pentanoylchlorid, Gehalt 85,7%) mit 9,39 g (39 mmol) einer frisch hergestellten Lösung von Chlorwasserstoff in Ethanol (15,15 Gew-%) versetzt. Das Gemisch wurde unter Stickstoff auf 50 °C erwärmt und bei dieser Temperatur 3,1 h gerührt. Anschliessend wurde auf 1°C abgekühlt und bei dieser Temperatur 1 h belassen. Das ausgefallene Produkt wurde abfiltriert, mit 10 ml eiskaltem Ethanol gewaschen und bei 40 °C/24 mbar getrocknet
- Ausbeute:: 4,05 g (58%) farblose Kristalle, Gehalt 98,3% (HPLC).

### Beispiel 2

### 2-Butyl-1,3-diaza-spiro[4.4]non-2-en-4-on-Monohydrochlorid

### (I, R¹ + R² = -(CH₂)₄-, R³ = n-Butyl)

Unter Stickstoff wurde innerhalb von 15 min bei 70 °C eine Lösung von 6,88 g (30 mmol) *N*-(1-Cyancyclopentyl)pentanamid (Gehalt 84,7%) zu einem Gemisch von 9,84 g (45 mmol) einer 16,7%igen Lösung von Chlorwasserstoff in Propanol und 11,71 g getrocknetem Propanol getropft, wobei ein Feststoff ausfiel. Das Gemisch wurde noch 1,7 bei 70 °C gerührt, auf 1 °C abgekühlt und 1 h bei dieser Temperatur belassen. Anschliessend wurde das Produkt abfiltriert, mit 10 ml eiskaltem Propanol gewaschen und bei 40 °C/24 mbar getrocknet.
Ausbeute: 5,74 g (79%), Gehalt 95,4% (titrimetrisch)
IR (KBr) 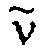 = 1779, 1642, 1517 cm⁻¹.
- ¹H NMR (DMSO-d₆): δ =: 13,64 (s, 2H); 2,80 (m, 2H), 1,7-2,0 (m, 10H); 1,34 (m, 2H), 0,91 (t, *J* = 7,3 Hz, 3H).
- ¹⁵N NMR (DMSO-d₆): δ =: -211,8; -219,6 (Standard: Acetanilid)

### Beispiel 3

### 2-Butyl-1,3-diaza-spiro[4.4]non-2-en-4-on-Monohydrochlorid

### (I, R¹ + R² = ―(CH₂)₄―, R³ = n-Butyl)

Unter Stickstoff wurden 6,88 g (30 mmol) *N*-(1-Cyancyclopentyl)pentanamid (Gehalt 84,7%) mit 27,3 g getrocknetem Isopropylalkohol und 9,92 g (39 mmol) einer frisch hergestellten 14,33%igen Lösung von Chlorwasserstoff in Isopropylalkohol 44 h bei Raumtemperatur gerührt. Anschliessend wurde das Gemisch auf 1 °C abgekühlt und ½ h bei dieser Temperatur belassen. Das ausgefallene Produkt wurde abfiltriert, mit 10 ml eiskaltem Isopropylalkohol gewaschen und bei 40 °C/24 mbar getrocknet.
- Ausbeute: 3,04 g (44%), Gehalt 99,7% (HPLC).

### Beispiel 4

### 2-Butyl-1,3-diaza-spiro[4.4]non-2-en-4-on-Monohydrochlorid

### (I, R¹ + R² ―(CH₂)₄―, R³ = n-Butyl)

Unter Stickstoff wurden 6,88 g (30 mmol) *N*-(1-Cyancyclopentyl)pentanamid (Gehalt 84,7%) mit 16,66 g getrocknetem Butanol und 10,06 g (39 mmol) einer frisch hergestellten 14,13%igen Lösung von Chlorwasserstoff in Butanol vorgelegt und unter Rühren zum Rückfluss erhitzt (vorgewärmtes Ölbad), wobei bereits beim Erreichen von ca. 100°C Innentemperatur Produkt ausfiel. Nach 2,9 h Rückfluss (115 °C) wurde das Gemisch auf 1 °C abgekühlt und 1 h bei dieser Temperatur belassen. Das ausgefallene Produkt wurde abfiltriert, mit 10 ml eiskaltem Butanol gewaschen und bei 40 °C/24 mbar getrocknet.
- Ausbeute:: 4,93 g (66%), Gehalt 92,7% (HPLC).

### Beispiel 5

### 2-Amino-2-methylbutannitril

In 78 ml Wasser wurden 40,0 g (800 mmol) Natriumcyanid gelöst. Zu dieser Lösung wurde bei Raumtemperatur unter Stickstoff eine Lösung von 47,5 g (880 mmol) Ammoniumchlorid in einem Gemisch von 70 ml konzentrierter wässriger Ammoniaklösung (0,92 mol NH₃) und 118 ml Wasser gegeben. Anschliessend wurde bei 20-25 °C (Wasserbad) ein Gemisch aus 51,8 g (714 mmol) Butanon und 76 ml Methanol (über Molekularsieb getrocknet) zugetropft. Das Reaktionsgemisch wurde noch ca. 2 h bei Raumtemperatur gerührt, dann auf 60 °C erwärmt und noch ca. 1 h auf dieser Temperatur gehalten. Nach dem Abkühlen wurde das Reaktionsgemisch einmal mit 200 ml und danach zweimal mit je 100 ml Dichlormethan extrahiert. Die vereinigten organischen Phasen wurden über 20 g Natriumsulfat getrocknet, filtriert und mit Dichlormethan auf 700 g Lösung verdünnt. Die so erhaltene Lösung wurde ohne weitere Aufarbeitung für die Acylierung verwendet.
- Ausbeute: 94% (GC).

### Beispiel 6

### N-(1-Cyan-1-methylpropyl)pentanamid

### (II, R¹ = Et, R² = Me, R³ = n-Butyl)

Zu 350 g einer Lösung von 2-Amino-2-methylbutannitril in Dichlormethan (aus Beispiel 5, max. 357 mmol) wurden unter Stickstoff bei Raumtemperatur 39,6 g (389 mmol) Triethylamin gegeben und dann bei 10-25 °C (Kühlung) innerhalb 1 h 47,9 g (389 mmol) Pentanoylchlorid zugetropft, wobei ein Feststoff (Triethylammoniumchlorid) ausfiel. Nach der Zugabe wurde noch 2 h bei Raumtemperatur gerührt. Dann wurde das Reaktionsgemisch mit 100 ml Wasser versetzt und die Phasen getrennt. Die organische Phase wurde mit 100 ml 1 N Salzsäure und dann mit 100 ml Wasser gewaschen, über 20 g Natriumsulfat getrocknet und schliesslich im Wasserstrahlvakuum eingeengt.
Ausbeute: 50,2 g Öl, Gehalt (GC) 86% (entspr. 66% d. Th.).
IR (Film). 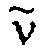 = 3305; 2230, 1656; 1535 cm⁻¹.
- ¹H NMR (DMSO-d₆) δ =: 8,13 (s, 1H); 2,14 (t, *J* = 7,3 Hz, 2 H); 1,8 (m, 2H); 1,52 (s, 3H); 1,4-1,5 (m, 2H); 1,29 (m, 2H); 0,98 (t, *J* = 7,3 Hz, 3H); 0,88 (t, *J* = 7,2 Hz, 3H)

### Beispiel 7

### 2-Butyl-4-ethyl-4-methyl-1H-imidazol-5(4H)-on-Hydrochlorid

### (I, R¹ = Et, R² = Me, R³ = n-Butyl, A⁻ = Cl⁻)

Unter Stickstoff wurden in einem Gemisch aus 25,6 g einer 16,7%igen Lösung von Chlorwasserstoff in Propanol (117 mmol HCl) und 53,8 g Propanol (über Molekularsieb getrocknet) 19,05 g (90 mmol) des *N*-(1-Cyan-1-methylpropyl)pentanamids aus Beispiel 6 bei 30 °C 6½ h gerührt Anschliessend wurde die so erhaltene klare gelbe Lösung über Nacht in den Kühlschrank gestellt. Die ausgefallenen Kristalle wurden abfiltriert, mit 10 ml eiskaltem Propanol gewaschen und bei 40 °C/24 mbar getrocknet.
Ausbeute: 5,38 g (27%) weisse Kristalle, Gehalt (HPLC) 99,5%.
IR (KBr): 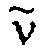 = 1779; 1638; 1519 cm⁻¹.
- ¹H NMR (DMSO-d₆). δ =: 13,71 (s, 1H); 2,87 (m, 2H); 1,7-1,8 (m, 4H); 1,43 (s, 3H); 1,37 (m, 2H); 0,92 (t, *J* = 7,4 Hz, 3H); 0,83 (t, *J* = 7,3 Hz, 3H).
- ¹⁵N NMR (DMSO-d6): δ =: -215,4;-217,7.

### Beispiel 8

### 2-Amino-2-phenylpropannitril

In 196 ml Methanol wurden unter Stickstoff 40,0 g (800 mmol) Natriumcyanid und 47,5 g (880 mmol) Ammoniumchlorid suspendiert. Anschliessend wurde bei Raumtemperatur innerhalb von 15 min ein Gemisch aus 87,5 g (714 mmol) Acetophenon und 76 ml Methanol (über Molekularsieb getrocknet) zugetropft. Das Reaktionsgemisch wurde noch 1 h bei Raumtemperatur gerührt, dann auf 40 °C erwärmt, bei dieser Temperatur 5½ h gehalten und noch 2 d bei 22 °C gerührt. Die so erhaltene orange Suspension wurde über eine Glasfritte filtriert, das Filtrat im Vakuum bei max. 35 °C auf ¼ des Volumens eingeengt und nochmals filtriert. Dieses Filtrat (ca. 100 g) wurde mit Diethylether auf 400 g verdünnt und ein weiteres Mal filtriert. Dann wurden in die so erhaltene klare orangerote Lösung innerhalb von 70 min 11,4 g (1,1 Äquivalente) Chlorwasserstoff eingeleitet, wobei ein heller Feststoff ausfiel Das Gemisch wurde über Nacht im Kühlschrank stehengelassen und dann der Überstand vom Niederschlag abdekantiert Der Niederschlag wurde mit 50 ml Diethylether gewaschen und in 100 ml Wasser gelöst. Die wässrige Lösung (pH ≈ 2,5) wurde mit konzentrierter Natronlauge auf pH 8,7 gestellt und dann mit 3 × 100 ml Diethylether extrahiert. Die vereinigten Etherextrakte wurden über 20 g Natriumsulfat getrocknet und dann im Vakuum eingedampft. Der Rückstand wurde zweimal mit je 15 ml Toluol aufgeschlämmt und wieder im Vakuum zur Trockne eingeengt.
Ausbeute: 56,2 g, Gehalt (¹H NMR) 83% (entspr 45% d. Th., bezogen auf Acetophenon).
IR (NaCl). 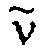 = 3378; 3313; 2224 cm⁻¹.
- ¹H NMR (CDCl₃): δ =: 7,63-7,68 (m, 2H); 7,30-7,43 (m, 3H); 2,08 (s, 2H); 1,74 (s, 3H).

### Beispiel 9

### N-(1-Cyan-1-phenylethyl)pentanamid

### (II, R¹ = Ph, R² = Me, R³ = n-Butyl)

Zu 55,8 g 2-Amino-2-phenylpropannitril (aus Beispiel 8, ca. 0,34 mol) und 38,1 g (375 mmol) Triethylamin in 280 g Dichlormethan wurden bei 12-23 °C (Kühlung) unter Stickstoff innerhalb von 55 min 46,1 g (375 mmol) Pentanoylchlorid getropft, wobei ein Feststoff (Triethylammoniumchlorid) ausfiel. Nach der Zugabe wurde noch 2 h bei Raumtemperatur gerührt. Dann wurde das Reaktionsgemisch mit 100 ml Wasser versetzt und die Phasen getrennt. Die organische Phase wurde mit 100 ml 1 N Salzsäure und dann mit 100 ml Wasser gewaschen, über 20 g Natriumsulfat getrocknet und schliesslich bei 50 °C im Wasserstrahlvakuum eingeengt. Zur Reinigung wurden 40,0 g des Rückstands (insgesamt 84,1 g beiger Feststoff) aus 230 ml siedendem Ethylacetat/Cyclohexan (70:30) umkristallisiert, nach dem Abkühlen auf Raumtemperatur über eine Glasfritte filtriert, mit 50 ml Cyclohexan gewaschen und bei 40 °C/30 mbar getrocknet.
Ausbeute: 31,71 g weisse Kristalle (umgerechnet auf die Gesamtmenge an Rohprodukt: 66,7 g, entspr. 84% d. Th., bezogen auf das Aminonitril).
IR (KBr) 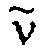 = 2228; 1657; 1539 cm⁻¹
¹H NMR (DMSO-d₆) δ = 7,3-7,4 (m, 5H); 7,03 (s, 1H); 2,15 (t, *J* =7,6 Hz, 2H); 1,77 (s, 3H); 1,55 (m, 2H); 1,30 (m, 2H); 0,89 (t, *J* = 7,3 Hz, 3H).

### Beispiel 10

### 2-Butyl-4-methyl-4-phenyl-1H-imidazol-5(4H)-on-Hydrochlorid

### (I, R¹ = Ph, R² = Me, R³ = n-Butyl, A⁻ = Cl⁻)

Unter Stickstoff wurden in einem Gemisch aus 9,2 g einer 15,5%igen Lösung von Chlorwasserstoff in Propanol (30 mmol HCl) und 33,3 g Propanol (über Molekularsieb getrocknet) 8,49 g (30 mmol) des *N*-(1-Cyan-1-phenylethyl)pentanamids aus Beispiel 9 auf 50 °C erwärmt und bei dieser Temperatur 3¼ h gerührt. Anschliessend wurde die so erhaltene klare gelbe Lösung über Nacht bei Raumtemperatur stehengelassen und dann bei 50 °C/16 mbar zur Trockne eingedampft. Der Rückstand (17,76 g) wurde bei Raumtemperatur in 30 ml Aceton 1¼ h aufgeschlämmt. Die Suspension wurde über eine Glasfritte filtriert, der Filterkuchen mit 10 ml Aceton gewaschen und bei 40 °C/24 mbar getrocknet.
Ausbeute 5,47 g weisser Feststoff, Gehalt (titrimetr.) 98% (entspr 67% d. Th, bezogen auf das Aminonitril)
IR (KBr). 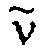 = 1787; 1633; 1517 cm⁻¹.
¹H NMR (DMSO-d₆): δ = 14,0 (s, 2H); 7,3-7,6 (m, 5H); 2,99 (t, J= 7,5 Hz, 2H); 1,7-2,0 (m, 2H); 1,85 (s, 3H); 1,38 (m, 2H); 0,93 (t, *J* = 7,5 Hz, 3H)
¹⁵N NMR (DMSO-d₆): δ = -213,5; -220,0.

## Patentansprüche

1. Verfahren zur Herstellung von 4-Oxoimidazoliniumsalzen der allgemeinen Formel 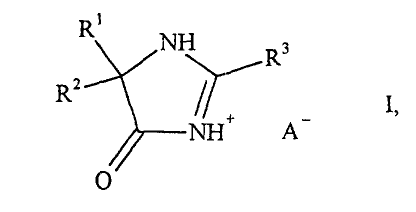
worin R¹ und R² entweder unabhängig voneinander C₁₋₁₀-Alkyl, C₂₋₁₀-Alkenyl, C₃₋₇-Cycloalkyl oder gegebenenfalls mit C₁₋₄-Alkyl, C₁₋₄-Alkoxy und Halogen substituiertes Aryl, Aryl-C₁₋₄-alkyl, Heteroaryl oder Heteroaryl-C₁₋₄-alkyl bedeuten, worin Aryl Phenyl oder Naphthyl und Heteroaryl Furyl oder Thienyl bedeutet,
oder R¹ und R² zusammen mit dem verbindenden Kohlenstoffatom einen drei- bis siebengliedrigen gesättigten oder ungesättigten carbocyclischen oder heterocyclischen Ring bilden,
R3 eine C₁₋₁₀-Alkylgruppe, C₂₋₁₀-Alkenylgruppe, C₃₋₇-Cycloalkylgruppe, eine gegebenenfalls mit C₁₋₄-Alkyl, C₁₋₄-Alkoxy und Halogen substituierte Arylgruppe, Aryl-C₁₋₄-Alkylgruppe oder Heteroarylgruppe ist, worin Aryl und Heteroaryl die obige Bedeutung haben,
und A⁻ ein Anion einer starken Säure aus der Gruppe bestehend aus den Halogenwasserstoffen, Schwefelsäure, Ameisensäure, Methansulfonsäure und Trifluoressigsäure ist,
dadurch gekennzeichnet, dass ein α-Acylamino-nitril der allgemeinen Formel 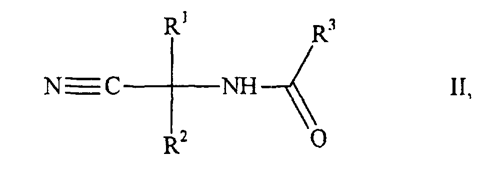 worin R¹, R² und R³ die oben gennanten Bedeutungen haben, in einem nichtwässrigen Lösungsmittel in Gegenwart eines niedrigen Alkohols aus der Gruppe Methanol, Ethanol, Propanol, Butanol oder Isopropylalkohol und einer straken Säure der Allgemeinen HA, worin A die oben gennante Bedeutung hat, cyclisiert wird.

2. Verfahren nach Anspruch 1, dadurch gekennzeichnet, dass als Säure HA Chlorwasserstoffsäure eingesetzt wird.

3. Verfahren nach Anspruch 1 oder 2, dadurch gekennzeichnet, dass als nichtwassriges Lösungsmittel ein Lösungsmittel aus der Gruppe der aromatischen Kohlenwasserstoffe oder aus der Gruppe der halogenierten Kohlenwasserstoffe eingesetzt wird.

4. Verfahren nach Anspruch 1 oder 2, dadurch gekennzeichnet, dass als niedriger Alkohol und nichtwässriges Lösungsmittel Methanol, Ethanol, Propanol, Butanol oder Isopropylalkohol eingesetzt wird.

5. Verfahren nach einem der Ansprüche 1 bis 4, dadurch gekennzeichnet, dass als α-Acylamino-nitril (II) eine Verbindung eingesetzt wird, in der R¹ und R² zusammen mit dem verbindenden Kohlenstoffatom einen Cyclopentan- oder Cyclohexanring bilden.

6. Verfahren nach einem der Ansprüche 1 bis 5, dadurch gekennzeichnet, dass als α-Acylamino-nitril (II) eine Verbindung eingesetzt wird, in der R³ eine C₁₋₆-Alkylgruppe ist

## Claims

1. Process for the preparation of 4-oxoimidazolinium salts corresponding to the general formula 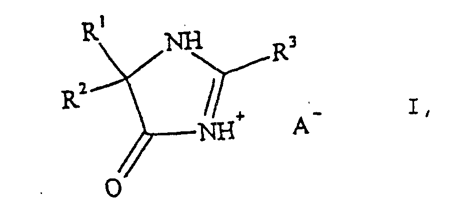
wherein R¹ and R² either, independently of one another, denote C₁₋₁₀-alkyl, C₂₋₁₀-alkenyl, C₃₋₇-cycloalkyl, or denote aryl, aryl-C₁₋₄-alkyl, heteroaryl or heteroaryl-C₁₋₄-alkyl which are optionally substituted with C₁₋₄-alkyl, C₁₋₄-alkoxy and halogen, wherein aryl denotes phenyl or naphthyl and heteroaryl denotes furyl or thienyl,
or R¹ and R² together with the linking carbon atom form a three- to six-membered saturated or unsaturated carbocyclic or heterocyclic ring,
R³ is a C₁₋₁₀-alkyl group, C₂₋₁₀-alkenyl group,
C₃₋₇-cycloalkyl group or is an aryl group, aryl-C₁₋₄-alkyl group or heteroaryl group which are optionally substituted with C₁₋₄-alkyl, C₁₋₄-alkoxy and halogen, wherein aryl and heteroaryl have the meanings given above,
and A⁻ is an anion of a strong acid selected from among the hydrogen halides, sulfuric acid, formic acid, methanesulfonic acid and trifluoroacetic acid, characterised in that an α-acylaminonitrile corresponding to the general formula 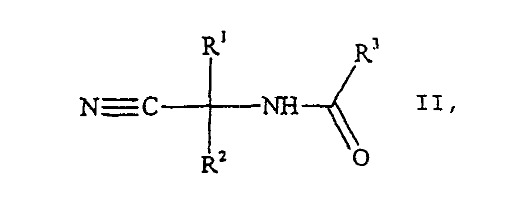 wherein R¹, R² and R³ have the meanings given above, is cyclised in a non-aqueous solvent in the presence of a low alcohol selected from methanol, ethanol, propanol, butanol or isopropyl alcohol and of a strong acid corresponding to the general formula HA, wherein A has the meaning given above.

2. Process according to claim 1, characterised in that hydrochloric acid is used as acid HA.

3. Process according to claim 1 or 2, characterised in that a solvent selected from the aromatic hydrocarbons or from the halogenated hydrocarbons is used as non-aqueous solvent.

4. Process according to claim 1 or 2, characterised in that methanol, ethanol, propanol, butanol or isopropyl alcohol is used as low alcohol and non-aqueous solvent.

5. Process according to one of claims 1 to 4, characterised in that the α-acylaminonitrile (II) used is a compound in which R¹ and R² together with the linking carbon atom form a cyclopentane ring or cyclohexane ring.

6. Process according to one of claims 1 to 5, characterised in that the α-acylaminonitrile (II) used is a compound in which R³ is a C₁₋₆-alkyl group.

## Revendications

1. Procédé de préparation de sels de 4-oxo-imidazolinium de formule générale 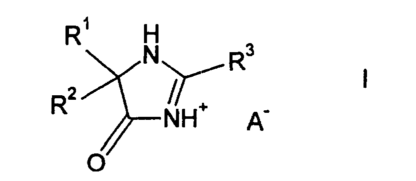
où R¹ et R² représentent indépendamment l'un de l'autre alkyle en C₁₋₁₀, alcényle en C₂₋₁₀, cycloalkyle en C₃₋₇ ou aryle, aryl-alkyle en C₁₋₄, hétéroaryle ou hétéroarylalkyle en C₁₋₄ éventuellement substitué par alkyle en C₁₋₄, alcoxy en C₁₋₄ et halogène, où aryle représente phényle ou naphtyle et hétéroaryle représente furyle ou thiényle,
ou bien R¹ et R² forment avec l'atome de carbone auquel ils sont liés un cycle carbocyclique ou hétérocyclique saturé ou insaturé à trois à sept chaînons,
R³ est un groupe alkyle en C₁₋₁₀, un groupe alcényle en C₂₋₁₀, un groupe cycloalkyle en C₃₋₇, un groupe aryle, un groupe aryl-alkyle en C₁₋₄ ou un groupe hétéroaryle éventuellement substitué par alkyle en C₁₋₄, alcoxy en C₁₋₄ et halogène, où aryle et hétéroaryle ont la signification ci-dessus,
et A⁻ est un anion d'un acide fort du groupe consistant en les hydrocarbures halogénés, l'acide sulfurique, l'acide formique, l'acide méthanesulfonique et l'acide trifluoroacétique,
caractérisé en ce qu'un α-acylaminonitrile de formule générale 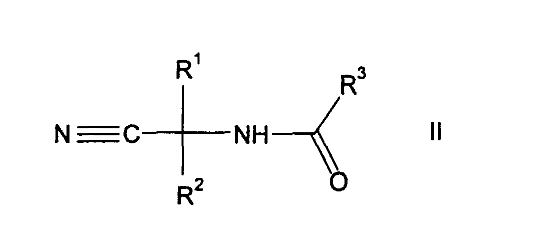 où R¹, R² et R³ ont les significations citées ci-dessus est cyclisé dans un solvant non aqueux en présence d'un alcool inférieur du groupe du méthanol, de l'éthanol, du propanol, du butanol et de l'isopropanol et d'un acide fort de formule générale HA où A a la signification citée ci-dessus.

2. Procédé selon la revendication 1 caractérisé en ce que l'acide chlorhydrique est utilisé comme acide HA.

3. Procédé selon la revendication 1 ou 2 caractérisé en ce qu'un solvant du groupe des hydrocarbures aromatiques ou du groupe des hydrocarbures halogénés est utilisé comme solvant non aqueux.

4. Procédé selon la revendication 1 ou 2 caractérisé en ce que le méthanol, l'éthanol, le propanol, le butanol ou l'isopropanol est utilisé comme alcool inférieur et solvant non aqueux.

5. Procédé selon l'une des revendications 1 à 4 caractérisé en ce qu'un composé dans lequel R¹ et R² forment avec l'atome de carbone auquel ils sont liés un cycle cyclopentane ou cyclohexane est utilisé comme α-acyl-aminonitrile (II).

6. Procédé selon l'une des revendications 1 à 5 caractérisé en ce qu'un composé dans lequel R³ est un groupe alkyle en C₁₋₆ est utilisé comme α-acylaminonitrile (II).
